# EUROPEAN PATENT SPECIFICATION

(11) **EP 2 059 270 B1**
(45) Date of publication and mention of the grant of the patent: **12.06.2013**
(21) Application number: 07794211.8
(22) Date of filing: 31.08.2007
(51) Int. Cl.: A61L 27/30, A61L 31/08, A61L 33/02

(54) **IMPLANT COATINGS HAVING IMPROVED HAEMOCOMPATIBILITY**
IMPLANTATBESCHICHTUNGEN MIT VERBESSERTER HÄMOKOMPATIBILITÄT
REVÊTEMENTS D'IMPLANTS POSSÉDANT UNE HÉMOCOMPATIBILITÉ AMÉLIORÉE

(30) Priority: 05.09.2006 US 842683 P
(43) Date of publication of application: 20.05.2009
(73) Proprietor: Doxa AB, 754 51 Uppsala (SE)
(72) Inventor: HERMANSSON, Leif, 260 42 Mölle (SE); PERSSON, Tobias, 753 17 Uppsala (SE); ENGQVIST, Håkan, 751 44 Knivsta (SE)
(74) Representative: Allee, Harriet Eva Charlotta
(86) International application number: PCT/SE2007/050606
(87) International publication number: WO 2008/030174

(56) References cited:
- WO-A-03/055449
- WO-A-2005/053764
- US-A1- 2006 134 160
- GERCKENS U ET AL: "Evaluation of a Tacrolimus-eluting coronary stent with nanoporous ceramic coating in treatment of native coronary artery lesions: Phase I and II of the present study" JOURNAL OF THE AMERICAN COLLEGE OF CARDIOLOGY, ELSEVIER, NEW YORK, NY, US, vol. 41, no. 6 Supplement A, 19 March 2003 (2003-03-19), page 7A, XP002381361 ISSN: 0735-1097

## Description

### TECHNICAL FIELD

For medical devices to be used in the blood system, the biocompatibility profile, with special reference to haemocompatibility, is of specific importance. Normally stents and coated stents to be used for implantation require complementary drugs in order for blood clotting behavior to be avoided. The present invention is directed to the use of Ca aluminate compositions for blood tissue implants, such as stents, which coating exhibits an improved stability in blood contact, with reduced amount of complementary drug or with no use of any complementary drugs or medication. The composition of the invention is especially intended for haemocompatible applications, and also for soft tissue applications, since both a direct blood contact and a soft tissue interaction is at hand. The invention also relates to a method of preparing the Ca aluminate coating on a blood tissue implant, coated implants obtainable by means of the method, and a method of implanting such coated blood tissue implant.

### STATE OF THE ART AND PROBLEM

Implants that are to interact with the human body should advantageously be composed of materials having a good biocompatibility. An example of such a material is the class known as "chemically bonded ceramics" (CBCs). The CBC materials are found as phosphates, aluminates, and silicates, all of which with calcium as main cation.

Examples of such materials are the high strength CBC materials, which are based on Ca-aluminates and/or Ca-silicates, such as those described in WO 2000/021489, WO 2001/076534, WO 2001/076535, WO 2004/037215, WO 2004/00239 and WO 2003/041662 and US 6,620,232 , US 6,969,424, US 7,025824. and US 7,048,792. Said materials have been proposed for use in dental applications as well as in orthopedic applications. The most frequently used chemically bonded ceramics are those based on Ca-phosphates, and blood clotting at the surface of these materials is generally been interpreted as a good sign for possible bone formation at the implant surface. A general description of ceramics used as biomaterials can be found in "An Introduction to Bioceramics", by L. Hench and J Wilson, World Scientific Publ. Co., Singapore, Hong-Kong.

The process that leads to thrombosis formation as blood contacts an artificial surface depends on a range of factors coupled to the material and its surface characteristics, the rheology and the biological aspects, commencing with the initial step of protein adsorption. The most widely used chemical compounds to reduce thrombogenecity and increase the haemocompatibility of stents are those based on phosphorylcholine (PC) or heparin substances. Heparin and PC can be coated upon the desired implant, but need some linking to the implant material. Heparin is often placed on top of another organic coating, e.g. polyamine (PAV). PC, on the other hand, is bound to the implant via linking of e.g. 3-amino-propyltrimethoxysilane (3-APTMS). Heparin based materials can also be delivered as a drug by systemic administration.

Heparin and PC also reduce the adhesion of bacteria. This is due to the chemistry of the compounds, which substantially reduces the tendency of protein adsorption.

A summary of the status for PC and Heparin-based materials is presented in a recent PhD thesis entitled "Characterization of surfaces designed for biomedical applications, by Emma Kristensen, Uppsala University, ISBN 91-554-6546-3, p 14-22 (2006).

To substantially reduce or avoid thrombogenecity of the surface of an implant it would be desirable to be able to use materials in direct blood contact not having any clotting tendency, thereby substantially reducing or avoiding the use of drugs, such as phosphorylcholine and heparin substances. Accordingly, it is an object of the present invention to improve the haemocompatibility of a blood tissue implant to such an extent as to substantially reduce or avoid the need of undesired medical treatment by medication. It is also an object to avoid the need of an additional linking substance.

### SUMMARY OF THE INVENTION

It has surprisingly been found that a coating layer as such, based on calcium aluminate compounds as a biomaterial, also exhibits the desired haemocompatibility and markedly reduced thrombogenecity. The coating can be applied directly to the substrate, without any additional linking substances as required.in the prior art. Also, the inventive coating does not require the use of any drugs and/or medication, as in the prior art. The coating material works as an improved surface of the blood tissue implant and at the same time as a preventing surface against blood clotting.

An additional feature of the invention is a good biocompatibility of the coating also towards soft tissue. Accordingly, a stent exposed to both blood and soft tissue in the human or animal body, exhibiting a coating of the invention will therefore have a good biocompatibility also to the soft tissue.

The resulting coating of hydrated Ca aluminate is stable, and loss of the coating material over time of use is virtually zero, in contrast to the conventionally used PC and heparin based coating materials.

Also, the hardness and strength of the inventive coating are considerably higher than for the prior art PC and heparin based coatings.

Moreover, the inventive coating can be applied by means of CVD and PVD, thus allowing for application of very thin layers, such as of about 1 to about 20 µm, and also for carefully controlling the thickness thereof.

Accordingly, in one aspect the present invention relates to the use of a powdered composition based on calcium aluminate and/or solid solution thereof forming a chemically bonded ceramic biomaterial on hydration, for forming a coating on metals, organic polymers and/or ceramics, to avoid or reduce thrombogenecity and/or adsorption of bacteria. Such use is set forth in claims 1-3.

In another aspect the present invention relates to an implant device to be in contact with the blood flow of a mammal, which device has a reduced thrombogenecity and adsorption of bacteria. Such devices are set forth in claims 9-16.

In yet an aspect the present invention relates to a method for preparing a device to be in contact with the blood flow of a mammal, wherein said device exhibits a reduced thrombogenecity and adsorption of bacteria. Such method is set out in claims 4-6.

Further aspects, advantages and details of the invention are described in the detailed description, Examples and in the claims.

### DETAILED DESCRIPTION OF THE INVENTION AND PREFERRED EMBODIMENTS .

For the purpose of the present disclosure the term "blood tissue implant" is intended to refer to an implant which is exposed to blood when implanted into the human or animal body, and more particularly a blood vessel implant. Blood in turn is considered a tissue of the human or animal body. Examples of blood tissue implant are stents, heart implants, e.g. heart valves, transfer implants into blood vessels, such as for dialysis machines, needles and long-term needles, and also grafts.

The surface of the implant to be coated may be made of metals, organic polymers and/or ceramics.

For compounds of the CaO-Al₂O₃-H₂O (CAH) system, it has surprisingly been found that the hydrated surfaces of a coating thereof react in a similar way found in PC and heparin based technology to avoid blood clotting. While not wishing to be bound to any specific theory it is believed that this is due to the partial dual charge characteristics of the hydrates formed in the CAH coatings, the wettability and the increased amount of water molecules just outside the implant surface, and that Ca-aluminate as a hydrate thus has a similar zwitter-ion mechanism as PC, which hinders the start of the cascade of coagulation. Therefore, water molecules will attach strongly to the surface due to the partial positive and negative charge, and proteins cannot reach the surface for anchoring. This contributes to reduced protein adsorption and possibly also to reduced adhesion of bacteria. The CAH system provides coatings with favorable general features with regard to adhesion to the substrate (high shear strength) and wear resistance. Favorable phases in the hydration process are katoite and gibbsite. These phases are formed at hydration processes above approximately 30°C.

General aspects of suitable Ca-Aluminate systems for use in the present invention are known in the prior art and have been described in e.g. US 7,048,792 and US 7,074,223.

The techniques used to apply the coating depend on the geometry of the device to be coated. For devices with relatively simple geometry CVD and PVD techniques can be used. For more complex geometries or extended devices sol-gel processing and precipitation techniques are preferred.

The coating can be applied to the implant device, such as a stent, or heart valve, in the form of a powder, or a solution/slurry thereof, into which the device conveniently can be immersed, as will be explained in more detail in the Examples.

In one embodiment the applied coating is completely hydrated before in vivo use, so that the desired katoite and gibbsite phases are formed before implantation.

In another embodiment of the invention the applied coating is only partially hydrated before *in vivo* use. This will favor the contact of the coating with the soft tissue, since the coating material will be more active and thus interaction with the tissue will occur. In this case a pH reducing agent may advantageously be added, since otherwise using partially unhydrated Ca-aluminate the pH may be undesirably high. The unhydrated Ca-aluminate phase immediately starts to hydrate and these hydrates show as the pre-hydrated Ca-aluminate surface increased haemocompatibility.

In a preferred embodiment the Ca-aluminate composition is (CaO)ₐ (Al₂O₃)_{b}, wherein the ratio a:b is in the interval from 0.5:1 to 3:1.

In a preferred embodiment the Ca-aluminate composition is 3CaO Al₂O₃ (C3A).

### EXAMPLE 1

Ca-aluminate (C3A) was synthesized at 1400°C in a conventional sintering furnace for 6 h. The material was crushed and thereafter milled for 72 h in a rotating mill using ceramics containers and with the milling media of Silicon nitride balls (15 mm in diameter) and iso-propanol as liquid. After thin film evaporation the powder was completely dried. The powder was poured into distilled water, to obtain a supersaturated solution. Implant devices of commercially pure titanium were dipped into the solution for different time periods. The solution was kept at 37°C. The layer thickness was qualitatively found to depend on the time of immersion, and was < 10 µm after 24 hrs. This layer was used in the evaluation in Example 3 below.

### EXAMPLE 2

In complementary studies the powder based on CaO Al₂O₃ (CA) - synthesized at 1375°C - was pressed to pellets and used as target in a PVD process, with the following features:
Size of the target: 10 x 10 cm
Density of the target: 60 % of theoretical density
PVD instrument: RF magnetron

Using the PVD process the Ca-aluminate target material was transferred to an implant device surface, in this case the surface of a thin Ti-plate, commercially pure titanium (1x4 mm). The thickness of the Ca-aluminate coating thus obtained was approximately 0.3 micrometer.

The coated surface was heat treated (i.e. hydrated) in water at 37°C and at 60°C. X-ray diffraction of the coating revealed the presence of katoite, 3CaO Al₂O₃ 6H₂O (C3AH6), and gibbsite, Al(OH)₃, as the only detectable phases. At temperatures below 30°C the unstable 8-phase, CaO 2Al₂O₃ 8H₂O (CA2H8), may be developed.

### EXAMPLE 3.

The coated devices obtained in Examples 1 and 2 were tested in a model system with human whole blood using a close circuit Chandler loop model. The model exposes the test material to fresh human whole blood. Reference material: Immobilized functional heparin. The loops are rotated at 33 rpm in a 37°C water bath for 60 minutes. After the incubation, the blood and the coating surfaces were investigated with special attention to clotting reactions. Blood samples were collected and supplemented with EDTA for cell count analysis. Blood from the loops was centrifuged to generate plasma for analysis of thrombin-antithrombin complex (TAT), C3a and TCC (Terminal complex of complement) complement marker. No or only limited clotting behaviour of the coatings were observed. Three test runs were performed with different blood donors. At each occasion, the material was tested in two loops, including a control loop without test material (control), making a total of 9 loops per test run. For each loop, a blood sample was immediately analysed in a cell counter. A plasma sample was prepared by centrifugation and immediately frozen to -70°C. A range of tests was performed on the blood/plasma. Also the donor blood was tested, without having passed the loop test (baseline). The collected blood samples and the coated surfaces were first evaluated with respect to clotting by the eye. The blood was positioned on a cloth to detect possible clots or contaminations from the test materials

The clotting behaviour and platelet count of the Ca-aluminate material was comparable to both control and baseline, showing very low clotting and maintaining high platelet count numbers, around 200 for the Ca aluminate coating and the references. The concentration of thrombin-antithrombin complex (TAT) was largely unaffected for the test procedure for the Ca-aluminate material. The Ca-aluminate material should not be expected to cause thrombotic complications in view of its very low tendency to induce activation of the coagulation system.

## Claims

1. Use of a coating on metals, organic polymers and/or ceramics formed from a chemically bonded ceramic biomaterial, which biomaterial is formed on hydration of a powdered composition based on calcium aluminate and/or a solid solution of calcium aluminate, to reduce or avoid thrombogenecity of the surface of said metals, organic polymers and/or ceramics and/or to reduce or avoid adsorption of bacteria to the surface of said metals, organic polymers and/or ceramics, said surface being an implant surface.

2. The use of claim 1, wherein the coating is formed on a blood tissue implant.

3. The use of claim 1 or 2, wherein the Ca-aluminate composition is (CaO)ₐ (Al₂O₃)_{b} wherein the ratio a/ b is in the interval 0.5 to 3, and preferably wherein the Ca-aluminate composition is 3CaO Al₂O₃ (C3A).

4. Method of preparing a coating on a blood tissue implant having reduced thrombogenecity reduced adsorption of bacteria comprising the steps of:
a) providing a blood tissue implant;
b) applying a coating of a powdered composition based on calcium aluminate and/or solid solution thereof forming a chemically bonded ceramic biomaterial on hydration thereof to a surface the implant; and
c) at least partially hydrating the coating formed in b) so as to form phases of katoite and/or gibbsite.

5. The method of claim 4, wherein in step b) a solution or slurry of the powdered composition is applied to a surface of the implant.

6. The method of claim 4, wherein in step b) the powder coating is applied using PVD, CVD or sol-gel processing.

7. The method of any one of claim 4-6, wherein in step c) the coating is hydrated in water-containing solution and/or in highly humid air (RH > 60 %) at temperatures above 30°C.

8. The method of any one of claim 4-7, wherein the coating in step b) is precipitated from Ca-aluminate water solutions at temperatures above 30°C.

9. A blood tissue implant exhibiting a coating based on calcium aluminate and/or a solid solution of calcium aluminate forming a chemically bonded ceramic biomaterial on hydration thereof, obtainable by means of the method of claim 5, wherein the coating after hydration exhibits a thickness of below 20 µm.

10. The blood tissue implant of claim 9, wherein the coating after hydration exhibits a thickness below 5 micrometer, and preferably a thickness below 1 micrometer.

11. The blood tissue implant of claim 9 or 10, wherein the coating contains the phases katoite and/or gibbsite.

12. The blood tissue implant of any one of the claims 9-11, wherein the coating containing the hydrated phases has crystal sizes below 100 nm.

13. The blood tissue implant of any one of the claims 9-12, wherein the coating is partially hydrated to a degree above 50%.

14. The blood tissue implant of any one of the claims 9-13, wherein the coating also contains a pH reducing agent.

15. The blood tissue implant of any one of the claims 9-14, including a complementary drug, preferably a complementary thrombogenecity reducing drug.

16. The blood tissue implant of any one of the claims 9-15, selected from the group consisting of grafts, stents, heart implants, transfer implants into blood vessels.

## Patentansprüche

1. Verwendung einer Beschichtung auf Metallen, organischen Polymeren und/oder Keramiken, ausgebildet aus einem chemisch gebundenen, keramischen Biomaterial, wobei dieses Biomaterial durch Hydration einer pulverartigen Zusammensetzung auf der Basis von Kalziumaluminat und/oder einer Feststofflösung von Kalziumaluminat ausgebildet wird, um die Thrombogenität der Oberfläche der Metalle, organischen Polymere und/oder Keramiken zu verringern oder zu vermeiden und/oder die Adsorption von Bakterien an der Oberfläche der Metalle, organischen Polymere und/oder Keramiken zu verringern oder zu vermeiden, wobei die Oberfläche eine Implantatoberfläche ist.

2. Verwendung nach Anspruch 1, bei der die Beschichtung auf einem Blutgewebeimplantat ausgebildet wird.

3. Verwendung nach Anspruch 1 oder 2, bei der die Ca-Aluminat-Zusammensetzung (CaO)ₐ(Al₂O₃)_{b} ist, wobei sich das Verhältnis a/b in dem Intervall 0,5 bis 3 befindet und die Ca-Aluminat-Zusammensetzung vorzugsweise 3CaO Al₂O₃ (C3A) ist.

4. Verfahren zum Vorbereiten einer Beschichtung auf einem Blutgewebeimplantat, das eine verringerte Thrombogenität und eine verringerte Adsorption von Bakterien hat, umfassend folgende Schritte:
a) Bereitstellen eines Blutgewebeimplantates;
b) Aufbringen einer Beschichtung einer pulverförmigen Zusammensetzung auf der Basis von Kalziumaluminat und/oder einer Feststofflösung desselben, die ein chemisch gebundenes, keramisches Biomaterial bei deren Hydrierung bilden, auf eine Oberfläche des Implantates; und
c) wenigstens teilweises Hydrieren der Beschichtung, die bei b) gebildet wird, um Phasen von Katoit und/oder Gibbsit zu bilden.

5. Verfahren nach Anspruch 4, bei dem bei Schritt b) eine Lösung oder Schlämme der pulverförmigen Zusammensetzung auf eine Oberfläche des Implantates aufgebracht wird.

6. Verfahren nach Anspruch 4, bei dem bei Schritt b) die Pulverbeschichtung mit Hilfe von PVD, CVD oder Sol-Gel-Verarbeitung aufgebracht wird.

7. Verfahren nach einem der Ansprüche 4 bis 6, bei dem bei Schritt c) die Beschichtung in einer wasserhaltigen Lösung und/oder in hochfeuchter Luft (RF > 60%) bei Temperaturen über 30°C hydriert wird.

8. Verfahren nach einem der Ansprüche 4 bis 7, bei dem die Beschichtung bei Schritt b) aus Ca-Aluminat-Wasserlösungen bei Temperaturen über 30°C ausfällt.

9. Blutgewebeimplantat, das eine Beschichtung auf der Basis von Kalziumaluminat und/oder einer Feststofflösung von Kalziumaluminat aufweist, die ein chemisch gebundenes, keramisches Biomaterial bei Hydrierung derselben bilden, beziehbar mit Hilfe des Verfahrens von Anspruch 5, wobei die Beschichtung nach der Hydrierung eine Dicke von weniger als 20 µm aufweist.

10. Blutgewebeimplantat nach Anspruch 9, bei dem die Beschichtung nach der Hydrierung eine Dicke unter 5 µm und vorzugsweise eine Dicke unter 1 µm aufweist.

11. Blutgewebeimplantat nach Anspruch 9 oder 10, bei dem die Beschichtung die Phasen Katoit und/oder Gibbsit beinhaltet.

12. Blutgewebeimplantat nach einem der Ansprüche 9 bis 11, bei dem die Beschichtung, die die hydrierten Phasen umfasst, Kristallgrößen unter 100 nm hat.

13. Blutgewebeimplantat nach einem der Ansprüche 9 bis 12, bei dem die Beschichtung teilweise bis zu einem Grad über 50% hydriert wird.

14. Blutgewebeimplantat nach einem der Ansprüche 9 bis 13, bei dem die Beschichtung zudem ein pH-Reduziertmittel enthält.

15. Blutgewebeimplantat nach einem der Ansprüche 9 bis 14, enthaltend ein Ergänzungsmedikament, vorzugsweise ein Thrombogenität verringerndes Ergänzungsmedikament.

16. Blutgewebeimplantat nach einem der Ansprüche 9 bis 15, das aus der Gruppe gewählt ist, die aus Transplantaten, Stents, Herzimplantaten und Transferimplantaten in Blutgefäße besteht.

## Revendications

1. Utilisation d'un revêtement sur des métaux, des polymères organiques et/ou des céramiques, formé à partir d'un biomatériau céramique chimiquement lié, lequel biomatériau est formé lors de l'hydratation d'une composition en poudre à base d'aluminate de calcium et/ou d'une solution solide d'aluminate de calcium, pour réduire ou éviter la thrombogénicité de la surface desdits métaux, polymères organiques et/ou céramiques, et/ou pour réduire ou éviter l'adsorption de bactéries à la surface desdits métaux, polymères organiques et/ou céramiques, ladite surface étant une surface d'un implant.

2. Utilisation selon la revendication 1, dans laquelle le revêtement est formé sur un implant de tissu sanguin.

3. Utilisation selon la revendication 1 ou 2, dans laquelle la composition à base d'aluminate de Ca est (CaO)ₐ(Al₂O₃)_{b} où le rapport a/b est situé dans l'intervalle de 0,5 à 3, et de préférence dans laquelle la composition d'aluminate de Ca est 3CaO Al₂O₃ (C3A).

4. Procédé pour préparer un revêtement sur un implant de tissu sanguin ayant une thrombogénicité réduite et une adsorption réduite des bactéries, comprenant les étapes consistant à :
a) fournir un implant de tissu sanguin ;
b) appliquer un revêtement d'une composition en poudre à base d'aluminate de calcium et/ou d'une solution solide de celui-ci formant un biomatériau céramique chimiquement lié lors de son hydratation à une surface de l'implant ; et
c) hydrater au moins partiellement le revêtement formé en b) de façon à former des phases de katoïte et/ou de gibbsite.

5. Procédé selon la revendication 4, dans lequel, dans l'étape b), une solution ou suspension de la composition en poudre est appliquée à une surface de l'implant.

6. Procédé selon la revendication 4, dans lequel, dans l'étape b), le revêtement en poudre est appliqué en utilisant un procédé PVD, CVD ou sol-gel.

7. Procédé selon l'une quelconque des revendications 4 à 6, dans lequel, dans l'étape c), le revêtement est hydraté dans une solution aqueuse et/ou dans de l'air très humide (HR > 60 %) à des températures supérieures à 30°C.

8. Procédé selon l'une quelconque des revendications 4 à 7, dans lequel le revêtement dans l'étape b) est précipité à partir de solutions aqueuses d'aluminate de Ca à des températures supérieures à 30°C.

9. Implant de tissu sanguin comportant un revêtement à base d'aluminate de calcium et/ou d'une solution solide d'aluminate de calcium formant un biomatériau céramique chimiquement lié lors de son hydratation, susceptible d'être obtenu au moyen du procédé de la revendication 5, dans lequel le revêtement, après hydratation, présente une épaisseur inférieure à 20 µm.

10. Implant de tissu sanguin selon la revendication 9, dans lequel le revêtement, après hydratation, présente une épaisseur inférieure à 5 micromètres, et de préférence une épaisseur inférieure à 1 micromètre.

11. Implant de tissu sanguin selon la revendication 9 ou 10, dans lequel le revêtement contient des phases de katoïte et/ou de gibbsite.

12. Implant de tissu sanguin selon l'une quelconque des revendications 9 à 11, dans lequel le revêtement contenant les phases hydratées a des tailles de cristal inférieures à 100 nm.

13. Implant de tissu sanguin selon l'une quelconque des revendications 9 à 12, dans lequel le revêtement est partiellement hydraté à un degré supérieur à 50 %.

14. Implant de tissu sanguin selon l'une quelconque des revendications 9 à 13, dans lequel le revêtement contient aussi un agent réducteur de pH.

15. Implant de tissu sanguin selon l'une quelconque des revendications 9 à 14, contenant un médicament complémentaire, de préférence un médicament complémentaire réduisant la thrombogénicité.

16. Implant de tissu sanguin selon l'une quelconque des revendications 9 à 15, choisi dans le groupe constitué par les greffes, les endoprothèses vasculaires, les implants cardiaques, les implants de transfert dans des vaisseaux sanguins.
